# EUROPÄISCHE PATENTSCHRIFT

(11) **EP 0 770 082 B1**
(45) Veröffentlichungstag und Bekanntmachung des Hinweises auf die Patenterteilung: **08.03.2000**
(21) Anmeldenummer: 95925797.3
(22) Anmeldetag: 30.06.1995
(51) Int. Cl.: C07D 495/04, A61K 31/435

(54) **DIOXO-THIOPYRANO-PYRIDIN-CARBONSÄURE-DERIVATE UND IHRE VERWENDUNG ALS ARZNEIMITTEL**
DIOXO-THIOPYRANO-PYRIDINE CARBOXYLIC ACID DERIVATIVES AND THEIR USE AS MEDICAMENTS
DERIVES D'ACIDE CARBOXYLIQUE DE DIOXO-THIOPYRANO-PYRIDINE ET LEUR UTILISATION COMME MEDICAMENTS

(30) Priorität: 13.07.1994 DE 4424678
(43) Veröffentlichungstag der Anmeldung: 02.05.1997
(73) Patentinhaber: BAYER AG, 51368 Leverkusen (DE)
(72) Erfinder: URBAHNS, Klaus, D-42115 Wuppertal (DE); HEINE, Hans-Georg, D-47800 Krefeld (DE); JUNGE, Bodo, D-42399 Wuppertal (DE); SCHOHE-LOOP, Rudolf, D-42327 Wuppertal (DE); SOMMERMEYER, Henning Dr., 51067 Köln (DE); GLASER, Thomas, D-51491 Overrath (DE); WITTKA, Reilinde, D-51069 Köln (DE); DE VRY, Jean-Marie-Viktor, D-51503 Rösrath (DE)
(86) Internationale Anmeldenummer: EP9502546
(87) Internationale Veröffentlichungsnummer: WO9602547

(56) Entgegenhaltungen:
- EP-A- 0 241 281
- CHEMICAL ABSTRACTS, vol. 108, no. 1, 4. Januar 1988, Columbus, Ohio, US; abstract no. 5876y, R. DUBURE ET AL '1,4-Dihydrobenzothieno(3,2-b)pyridine 5,5-dioxides' Seite 558 ; & KHIM. GETEROTSIKL. SOEDIN, Nr.11, 1986 Seiten 1563 - 1567

## Beschreibung

Die vorliegende Erfindung betrifft Dioxo-thiopyrano-pyridin-carbonsäure-derivate, ein Verfahren zu ihrer Herstellung und ihre Verwendung als Arzneimittel, insbesondere als cerebral wirksame Mittel.

Cyclische Sulfondihydropyridine sind bereits bekannt [vgl. J. Heterocycl. Chem. 27 (5), 1453-6, 1990].

EP-A-0 241 281 offenbart Thiacycloalkenopyridine mit calciumantagonistischer Wirkung.

Die vorliegende Erfindung betrifft neue 1,1-Dioxo-2H-thiopyrano-[2,3-b]pyridin-7-carbonsäure-derivate und -ester der allgemeinen Formel (I), 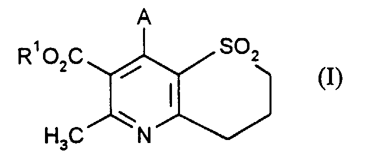 in welcher
- A: für Aryl mit 6 bis 10 Kohlenstoffatomen oder Pyridyl steht, die gegebenenfalls bis zu 3-fach gleich oder verschieden durch Nitro, Cyano, Cycloalkyl mit 3 bis 7 Kohlenstoffatomen, Halogen, Trifluormethyl oder durch geradkettiges oder verzweigtes Alkylthio, Alkyl oder Alkoxy mit jeweils bis zu 6 Kohlenstoffatomen substituiert sind,
- R¹: für Wasserstoff oder für geradkettiges oder verzweigtes Alkyl mit bis zu 8 Kohlenstoffatomen steht,
und deren Salze.

Im Rahmen der Erfindung sind physiologisch unbedenkliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für Phenyl, Naphthyl oder Pyridyl steht, die gegebenenfalls bis zu 3-fach gleich oder verschieden durch Nitro, Cyano, Fluor, Chlor, Brom, Iod, Cyclopropyl, Cyclopentyl, Cyclohexyl, Trifluormethyl oder durch geradkettiges oder verzweigtes Alkylthio, Alkyl oder Alkoxy mit jeweils bis zu 4 Kohlenstoffatomen substituiert sind,
- R¹: für Wasserstoff oder für geradkettiges oder verzweigtes Alkyl mit bis zu 6 Kohlenstoffatomen steht,
und deren Salze.

Besonders bevorzugt sind Verbindungen der allgemeinen Formel (I),
in welcher
- A: für Phenyl oder Pyridyl steht, die gegebenenfalls bis zu 3-fach gleich oder verschieden durch Nitro, Cyano, Fluor, Chlor, Brom, Iod, Trifluormethyl, Cyclohexyl, Methyl, Methoxy oder durch Methylthio substituiert sind,
- R¹: für Wasserstoff oder für geradkettiges oder verzweigtes Alkyl mit bis zu 4 Kohlenstoffatomen steht,
und deren Salze.

Außerdem wurde ein Verfahren zur Herstellung der erfindungsgemäßen Verbindungen der allgemeinen Formel (I) gefunden, dadurch gekennzeichnet, daß man
Aldehyde der allgemeinen Formel (II)

   A-CHO (II)

   in welcher
A die oben angegebene Bedeutung hat,
mit Verbindungen der allgemeinen Formel (III) 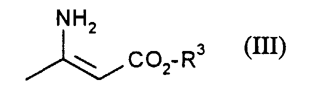 in welcher
R³ die oben angegebene Bedeutung von R¹ hat, aber nicht für Wasserstoff steht,
und Tetrahydrothiopyran-3-on-1,1-dioxid,
in inerten Lösemitteln zunächst zu den Verbindungen der allgemeinen Formel (IV) 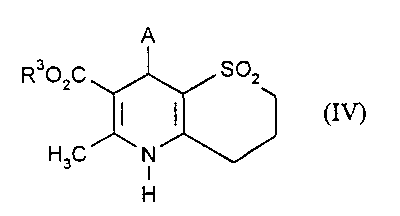 in welcher
A und R³ die oben angegebene Bedeutung haben
   umsetzt,
und in einem zweiten Schritt eine Oxidation in inerten Lösemitteln durchführt,
und im Fall der Säuren (R¹ = H) die Ester verseift.

Das erfindungsgemäße Verfahren kann durch folgendes Formelschema beispielhaft erläutert werden:

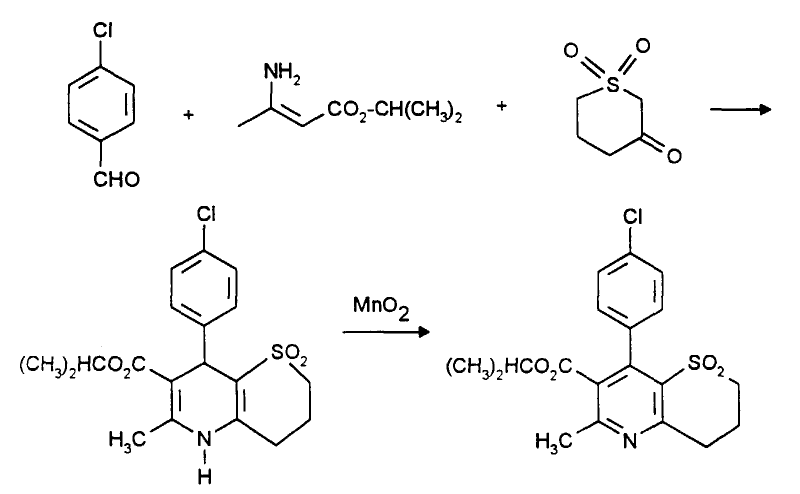

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt ist Ethanol.

Die Reaktionstemperaturen können in einem größeren Bereich variiert werden. Im allgemeinen arbeitet man zwischen +10°C und +150° C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für die Oxidation eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt ist Methylenchlorid.

Als Oxidationsmittel eignen sich im allgemeinen 2,3-Dichlor-4,5-dicyan-p-benzochinon und Derivate, Pyridiniumdichromat, elementares Brom, Jod und Mangandioxid. Bevorzugt ist Mangandioxid.

Das Oxidationsmittel wird im allgemeinen in einer Menge von 1 mol bis 20 mol, bevorzugt von 1 mol bis 5 mol, bezogen auf 1 mol der Verbindungen der allgemeinen Formel (IV) eingesetzt.

Die Reaktionstemperaturen können in einem größeren Bereich variiert werden. Im allgemeinen arbeitet man zwischen +10°C und +150°C, vorzugsweise zwischen -20°C und +100°C, insbesondere im Siedebereich des verwendeten Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Enantiomerenreine Formen erhält man z.B., indem man Diastereomerengemische der Verbindungen der allgemeinen Formel (I), in welcher R¹ für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formeln (II) und (III) sind an sich bekannt.

Die Verbindungen der allgemeinen Formel (IV) sind teilweise bekannt oder können beispielsweise wie oben beschrieben hergestellt werden.

Die erfindungsgemäßen Verbindungen der allgemeinen Formel (I) zeigen ein nicht vorhersehbares, wertvolles pharmakologisches Wirkspektrum.

Sie sind Modulatoren mit Selektivität für Calciumabhängige Kalium-Kanäle großer Leitfähigkeit (BK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie Demenzen beispielsweise Multiinfarktdemenz (MID), primär degenerativer Demenz (PDD), präsenile und senile Demenz vom Typ der Alzheimerschen Krankheit, HIV-Demenz und andere Demenzformen, Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung der Folgen cerebraler Durchblutungsstörungen wie cerebraler Ischämien, Schlaganfällen, Schädel-Hirn-Traumata und Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüberhinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina und Diabetes.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellen in 24-well-Kulturschalen kultiviert; am Versuchstag wird das Kulturmedium entfernt, und die Zellen werden gründlich mit Inkubationsmedium, bestehend aus Hepes-gepufferter Salzlösung (HBS, Hepes 20 mM, NaCl 150, KCl 5,4, CaCl₂ 1,8, MgCl₂ 0,8, NaHPO₄ 0,84, Glucose 5,5) gewaschen. Anschließend werden die Zellen für 60 min mit ⁸⁶Rubidium (⁸⁶Rb, 0.25µCi/well) in Inkubationsmedium beladen. Nach Absaugen der ⁸⁶Rb-Lösung und Waschen der Zellen wird zur Stimulation des ⁸⁶Rb-Effluxes 1 µM Ionomycin in Ab- und Anwesenheit von Prüfsubstanz in Inkubationspuffer (Gesamtvolumen 0.5 ml) zupipettiert und 10 min bei 37°C inkubiert. Zur Bestimmung des Effluxes wird der Überstand in Zählfläschchen überführt. Zur Bestimmung des noch verbliebenen intrazellulären ⁸⁶Rb werden die Zellen 30 min mit 0,5 M NaOH / 0.1% Triton · 100 behandelt. Anschließend wird die Lösung in Zählfläschchen überführt. Die in den Zählfläschchen enthaltene Menge an ⁸⁶Rb wird im Flüssigszintillationszähler bestimmt. Aus den Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Laufmittelgemische:

- a :: Methylenchlorid / AcOEt 10+1
- b :: Methylenchlorid / MeOH 10+1
- c :: PE / AcOEt 7+3
- d :: PE / AcOEt 1+1

### Ausgangsverbindungen

### Beispiel I

3,4,5,8-Tetrahydro-8-(4-chlorphenyl)-1,1-dioxo-2H-thiopyrano-[2,3-b]-pyridin-7-carbonsäureisopropylester

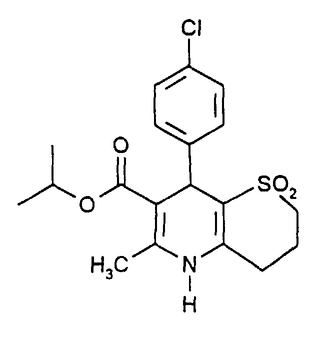

2.40 g (20 mmol) 4-Chlorbenzaldehyd, 4,86 g (20 mmol) Aminocrotonsäureisopropylester und 2,96 g (20 mmol) Tetrahydrothiopyran-3-on-1,1-dioxid werden 3 h in Ethanol bei Rückfluß gekocht. Der ausgefallene Feststoff wird abgesaugt und mit Ethanol gewaschen (5 g, 63 %).

### Herstellungsbeispiele

### Beispiel 1

3,4-Dihydro-8-(4-chlorphenyl)-1,1-dioxo-2H-thiopyrano-[2,3-b]-pyridin-7-carbonsäureisopropylester

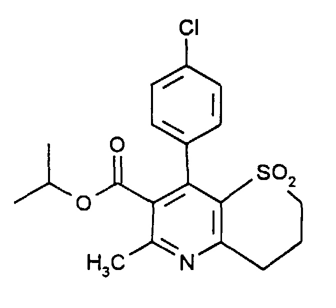

2.0 g (5,0 mmol) der Verbindung aus Beispiel I werden in 150 ml CH₂Cl₂ gelöst und mit 7,0 g MnO₂ versetzt. Man hält 2h unter Rückfluß, saugt über Celite ab und engt ein. Der Rückstand kristallisiert aus Ether/Petrolether. Man erhält 1,77 g (90%), (MS: 393), R_{f} (b) 0,86.

In Analogie zur Vorschrift des Beispiels 1 werden die in Tabelle 1 aufgeführten Verbindungen hergestellt:

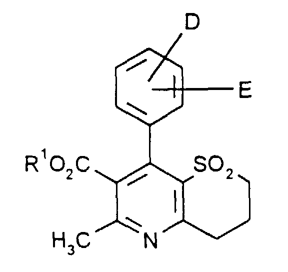

| **Bsp.-Nr.** | **R**^{**1**} | **D** | **E** | **R**_{**f**}^{*****} | **Ausbeute (% d.Th.)** |
|---|---|---|---|---|---|
| 2 | -CH₃ | 4-Cl | 3-H | 0,73 (b) | 77 |
| 3 | -CH₃ | 2-Cl | 3-Cl | 0,79 (b) | 68 |
| 4 | -CH(CH₃)₂ | 2-Cl | 3-Cl | 0,71 (b) | 87 |

## Patentansprüche

1. Dioxo-thiopyrano-pyridin-carbonsäure-derivate der allgemeinen Formel (I) 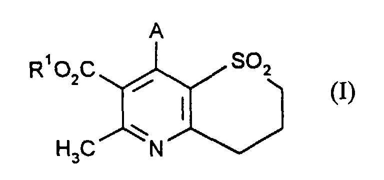 in welcher
A für Aryl mit 6 bis 10 Kohlenstoffatomen oder Pyridyl steht, die gegebenenfalls bis zu 3-fach gleich oder verschieden durch Nitro, Cyano, Cycloalkyl mit 3 bis 7 Kohlenstoffatomen, Halogen, Trifluormethyl oder durch geradkettiges oder verzweigtes Alkylthio, Alkyl oder Alkoxy mit jeweils bis zu 6 Kohlenstoffatomen substituiert sind,
R¹ für Wasserstoff oder für geradkettiges oder verzweigtes Alkyl mit bis zu 8 Kohlenstoffatomen steht,
und deren Salze.

2. Dioxo-thiopyrano-pyridin-carbonsäure-Derivate nach Anspruch 1
wobei
A für Phenyl, Naphthyl oder Pyridyl steht, die gegebenenfalls bis zu 3-fach gleich oder verschieden durch Nitro, Cyano, Fluor, Chlor, Brom, Iod, Trifluormethyl, Cyclopropyl, Cyclopentyl, Cyclohexyl oder durch geradkettiges oder verzweigtes Alkylthio, Alkyl oder Alkoxy mit jeweils bis zu 4 Kohlenstoffatomen substituiert sind,
R¹ für Wasserstoff oder für geradkettiges oder verzweigtes Alkyl mit bis zu 6 Kohlenstoffatomen steht,
und deren Salze.

3. Dioxo-thiopyrano-pyridin-carbonsäure-derivate nach Anspruch 1
wobei
A für Phenyl oder Pyridyl steht, die gegebenenfalls bis zu 3-fach gleich oder verschieden durch Nitro, Cyano, Fluor, Chlor, Brom, Iod, Trifluormethyl, Cyclohexyl, Methyl, Methoxy oder durch Methylthio substituiert sind,
R¹ für Wasserstoff oder für geradkettiges oder verzweigtes Alkyl mit bis zu 4 Kohlenstoffatomen steht,
und deren Salze.

4. Dioxo-thiopyrano-pyridin-carbonsäure-derivate nach Anspruch 1 bis 3 zur therapeutischen Anwendung.

5. Verfahren zur Herstellung von Dioxo-thiopyrano-pyridin-carbonsäure-derivaten nach Anspruch 1 bis 3
dadurch gekennzeichnet, daß man
Aldehyde der allgemeinen Formel (II)
A-CHO (II)
in welcher
A die oben angegebene Bedeutung hat,
mit Verbindungen der allgemeinen Formel (III) 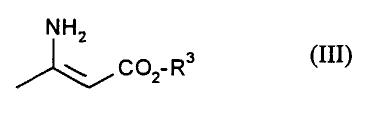 in welcher
R³ die oben angegebene Bedeutung von R¹ hat, aber nicht für Wasserstoff steht,
und Tetrahydrothiopyran-3-on-1,1-dioxid,
in inerten Lösemitteln zunächst zu den Verbindungen der allgemeinen Formel (IV) 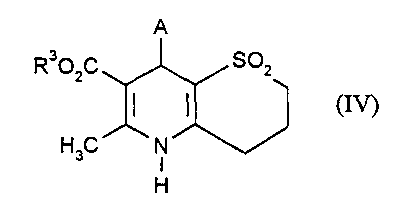 in welcher
A und R³ die oben angegebene Bedeutung haben
umsetzt,
und in einem zweiten Schritt eine Oxidation in inerten Lösemitteln durchführt,
und im Fall der Säuren ( R¹ = H) die Ester verseift.

6. Verfahren nach Anspruch 5, dadurch gekennzeichnet, daß als Oxidationsmittel Mangandioxid eingesetzt wird.

7. Arzneimittel enthaltend mindestens ein Dioxo-thiopyrano-pyridin-carbonsäure-derivat nach Anspruch 1 bis 3.

8. Arzneimittel nach Anspruch 7 zur selektiven Modulation von calciumabhängigen Kalium-Kanälen mit großer Leitfähigkeit.

9. Verwendung von Dioxo-thiopyrano-pyridin-carbonsäure-derivaten nach Anspruch 1 bis 3 zur Herstellung von Arzneimitteln.

10. Verwendung nach Anspruch 9 zur Herstellung von selektiven Modulatoren für calciumabhängige Kalium-Kanäle mit großer Leitfähigkeit.

## Claims

1. Dioxo-thiopyrano-pyridine-carboxylic acid derivatives of the general formula (I) 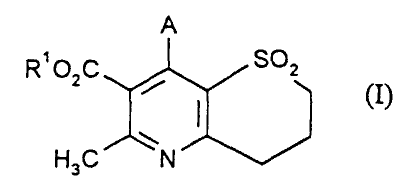 in which
A represents aryl having 6 to 10 carbon atoms, or pyridyl, each of which is optionally substituted up to 3 times by identical or different substituents from the group consisting of nitro, cyano, cycloalkyl having 3 to 7 carbon atoms, halogen and trifluoromethyl or by straight-chain or branched alkylthio, alkyl or alkoxy in each case having up to 6 carbon atoms,
R¹ represents hydrogen or straight-chain or branched alkyl having up to 8 carbon atoms,
and their salts.

2. Dioxo-thiopyrano-pyridine-carboxylic acid derivatives according to Claim 1
where
A represents phenyl, naphthyl or pyridyl, each of which is optionally substituted up to 3 times by identical or different substituents from the group consisting of nitro, cyano, fluorine, chlorine, bromine, iodine, trifluoromethyl, cyclopropyl, cyclopentyl and cyclohexyl or by straight-chain or branched alkylthio, alkyl or alkoxy in each case having up to 4 carbon atoms,
R¹ represents hydrogen or straight-chain or branched alkyl having up to 6 carbon atoms,
and their salts.

3. Dioxo-thiopyrano-pyridine-carboxylic acid derivatives according to Claim 1
in which
A represents phenyl or pyridyl, each of which is optionally substituted up to 3 times by identical or different substituents from the group consisting of nitro, cyano, fluorine, chlorine, bromine, iodine, trifluoromethyl, cyclohexyl, methyl and methoxy or by methylthio,
R¹ represents hydrogen or straight-chain or branched alkyl having up to 4 carbon atoms,
and their salts.

4. Dioxo-thiopyrano-pyridine-carboxylic acid derivatives according to Claims 1 to 3 for therapeutic use.

5. Process for the preparation of dioxo-thiopyranopyridine-carboxylic acid derivatives according to Claims 1 to 3
characterized in that
aldehydes of the general formula (II)
A-CHO (II)
in which
A has the abovementioned meaning,
are reacted with compounds of the general formula (III) 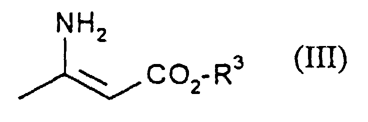 in which
R³ has the abovementioned meaning of R¹, but does not represent hydrogen,
and tetrahydrothiopyran-3-one-1,1-dioxide,
in inert solvents first to give the compounds of the general formula (IV) 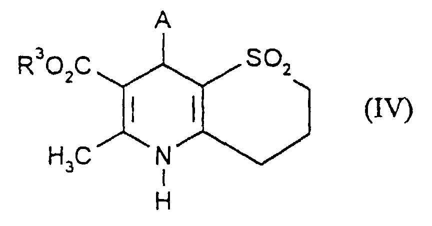 in which
A and R³ have the abovementioned meaning,
and in a second step an oxidation is carried out in inert solvents,
and in the case of the acids (R¹ = H) the esters are hydrolysed.

6. Process according to Claim 5, characterized in that manganese dioxide is employed as an oxidizing agent.

7. Medicaments containing at least one dioxo-thiopyrano-pyridine-carboxylic acid derivative according to Claims 1 to 3.

8. Medicaments according to Claim 7 for the selective modulation of calcium-dependent potassium channels of high conductivity.

9. Use of dioxo-thiopyrano-pyridine-carboxylic acid derivatives according to Claims 1 to 3 for the production of medicaments.

10. Use according to Claim 9 for the production of selective modulators for calcium-dependent potassium channels of high conductivity.

## Revendications

1. Dérivés de l'acide dioxo-thiopyranno-pyridine-carboxylique, de formule générale (I) : 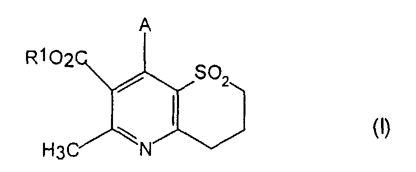 dans laquelle :
A représente aryle ayant 6 à 10 atomes de carbone ou pyridyle, qui sont facultativement substitués jusqu'à 3 fois, de manière identique ou différente, par nitro, cyano, cycloalcoyle ayant 3 à 7 atomes de carbone, halogène, trifluorométhyle ou par alcoylthio, alcoyle ou alcoxy linéaires ou ramifiés, ayant chaque fois jusqu'à 6 atomes de carbone,
R¹ représente hydrogène ou alcoyle linéaire ou ramifié ayant jusqu'à 8 atomes de carbone,
et leurs sels.

2. Dérivés de l'acide dioxo-thiopyranno-pyridine-carboxylique suivant la revendication 1, où
A représente phényle, naphtyle ou pyridyle, qui sont facultativement substitués jusqu'à 3 fois, de manière identique ou différente, par nitro, cyano, fluor, chlore, brome, iode, trifluorométhyle, cyclopropyle, cyclopentyle, cyclohexyle ou par alcoylthio, alcoyle ou alcoxy linéaires ou ramifiés, ayant chaque fois jusqu'à 4 atomes de carbone,
R¹ représente hydrogène ou alcoyle linéaire ou ramifié ayant jusqu'à 6 atomes de carbone,
et leurs sels.

3. Dérivés de l'acide dioxo-thiopyranno-pyridine-carboxylique suivant la revendication 1, où
A représente phényle ou pyridyle, qui sont facultativement substitués jusqu'à 3 fois, de manière identique ou différente, par nitro, cyano, fluor, chlore, brome, iode, trifluorométhyle, cyclohexyle, méthyle, méthoxy ou méthylthio,
R¹ représente hydrogène ou alcoyle linéaire ou ramifié ayant jusqu'à 4 atomes de carbone,
et leurs sels.

4. Dérivés de l'acide dioxo-thiopyranno-pyridine-carboxylique suivant les revendications 1 à 3, pour une utilisation thérapeutique.

5. Procédé de préparation de dérivés de l'acide dioxo-thiopyranno-pyridine-carboxylique suivant les revendications 1 à 3, caractérisé en ce que l'on fait réagir
un aldéhyde de formule générale (II) :
A-CHO (II)
dans laquelle
A a la signification donnée ci-dessus, avec un composé de formule générale (III) : 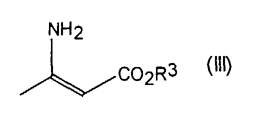 dans laquelle :
R³ a la signification donnée ci-dessus pour R¹, mais ne représente pas hydrogène,
et le tétrahydrothiopyrann-3-one-1,1-dioxyde,
dans un solvant inerte, d'abord pour obtenir un composé de formule générale (IV) : 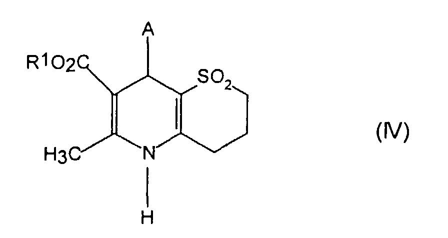 dans laquelle :
A et R³ ont la signification donnée ci-dessus,
et dans une deuxième étape, on réalise une oxydation dans un solvant inerte,
et dans le cas des acides (R¹ = H), on saponifie l'ester.

6. Procédé suivant la revendication 5, caractérisé en ce que l'on met en oeuvre du dioxyde de manganèse comme agent d'oxydation.

7. Médicament contenant au moins un dérivé de l'acide dioxo-thiopyranno-pyridine-carboxylique suivant les revendications 1 à 3.

8. Médicament suivant la revendication 7, pour la modulation sélective des canaux potassiques dépendant du calcium pour une plus grande conductibilité.

9. Utilisation des dérivés de l'acide dioxo-thiopyranno-pyridine-carboxylique suivant les revendications 1 à 3, dans la préparation de médicaments.

10. Utilisation suivant la revendication 9, dans la préparation de modulateurs sélectifs des canaux potassiques dépendant du calcium pour une plus grande conductibilité.
